# EUROPEAN PATENT SPECIFICATION

(11) **EP 2 316 449 B1**
(45) Date of publication and mention of the grant of the patent: **02.10.2013**
(21) Application number: 09804992.7
(22) Date of filing: 05.08.2009
(51) Int. Cl.: A61K 31/375, A61K 9/16, A61K 9/20, A61K 9/28, A61K 9/48, A61K 9/50, A61K 31/195, A61K 47/02, A61K 47/12, A61K 47/22, A61P 17/00

(54) **STABLE PHARMACEUTICAL COMPOSITION CONTAINING TRANEXAMIC ACID AND ASCORBIC ACID**
STABILE PHARMAZEUTISCHE ZUSAMMENSETZUNG MIT TRANEXAMSÄURE UND ASCORBINSÄURE
COMPOSITION PHARMACEUTIQUE STABLE CONTENANT DE L'AIDE TRANEXAMIQUE ET DE L'ACIDE ASCORBIQUE

(30) Priority: 06.08.2008 JP 2008203127
(43) Date of publication of application: 04.05.2011
(73) Proprietor: Daiichi Sankyo Healthcare Co., Ltd., Chuo-ku Tokyo 103-8541 (JP)
(72) Inventor: MAFUNE, Eiichi, Chuo-ku Tokyo 103-8541 (JP); MATSUSHITA, Takeshi, Chuo-ku Tokyo 103-8541 (JP); MURAKAMI, Toshio, Chuo-ku Tokyo 103-8541 (JP); MOCHIZUKI, Yusuke, Chuo-ku Tokyo 103-8541 (JP); SHIMADA, Kimiyoshi, Toyama-shi Toyama 939-8221 (JP); TSUJII, Ken, Toyama-shi Toyama 939-8221 (JP)
(74) Representative: Oldroyd, Richard Duncan
(86) International application number: PCT/JP2009/063844
(87) International publication number: WO 2010/016509

(56) References cited:
- EP-A2- 1 068 868
- WO-A1-2006/003965
- JP-A- 3 279 313
- JP-A- 9 286 726
- JP-A- 2004 091 473
- JP-A- 2004 217 655

## Description

The present invention relates to a pharmaceutical composition which is less likely to cause discoloration even if tranexamic acid and ascorbic acid are allowed to uniformly exist in the same composition without separating tranexamic acid and ascorbic acid from each other by a boundary or the like. The invention also relates to a solid pharmaceutical preparation for oral administration comprising the pharmaceutical composition.

### [Background Art]

Tranexamic acid has anti-bleeding (as an antiplasmin), antiallergic and antiinflammatory effects and the like, is widely used as ethical drugs and is blended also in OTC drugs. After it was reported in 1979 that when tranexamic acid was administered to a patient with chronic urticaria, chloasma which happened to occur at the same time in the same patient disappeared, tranexamic acid began to be prescribed for the treatment of chloasma (see, for example, NPL 1).

It has been known since long time ago that ascorbic acid suppresses the production of melanin pigment, and also that due to the action of degrading melanin pigment which has been already accumulated, ascorbic acid suppresses pigmentation. As for the OTC drugs, in the approved indications of preparations comprising vitamin C as a base component, blots, freckles, pigmentation caused by sunburn or rash are included (see NPL 2).

Further, a preparation for treatment of pigmentation comprising tranexamic acid and ascorbic acid has been disclosed, and is reported to show a superior degree of improvement of pigmentation than the case where tranexamic acid or ascorbic acid is administered as a single drug (see PTL 1).

Further, a skin whitening composition comprising tranexamic acid, ascorbic acid and L-cysteine has been disclosed by the present inventors, and it has been known that pigmentation is further suppressed than the concomitant use of tranexamic acid and ascorbic acid (see PTL 2). Further, in Preparation Examples 2.3 of Patent Document 2, a skin whitening composition comprising tranexamic acid, ascorbic acid, L-cysteine and tocopherol is also disclosed.

A solid pharmaceutical composition for oral administration in which an organic acid or an antioxidant other than ascorbic acid or tocopherol is further added to a pharmaceutical composition comprising tranexamic acid and ascorbic acid has not yet been known so far.

### [Citation List]

### [Patent Literature]

[PTL 1]
   Japan Laid Open Patent Publication No. 1992-243825
[PTL 2]
   Japan Laid Open Patent Publication No. 2004-217655

### [Non Patent Literature]

[NPL 1]
   Pharmacia Vol. 44, No. 5, 2008, pp. 437-442
[NPL 2]
   Ippan-yo Iyakuhin Seizo (Yunyu) Shonin Kijun (Approval Standards for Manufacturing (Import) of Non-Prescription Drugs), Jiho, Inc., 2008

### [Summary of Invention]

### [Problem to Be Solved]

The present inventors actually produced, on a trial basis, a granule formulation comprising tranexamic acid and ascorbic acid disclosed in Example 1 of PTL 1 and recognized a problem that the formulation gradually changed its color (red discoloration) when it was subjected to a long term storage (in an accelerated test at a temperature of 40°C and a relative humidity of 75%). Therefore, the present inventors hypothesized that it is necessary that, in a preparation comprising tranexamic acid and ascorbic acid, tranexamic acid and ascorbic acid be not brought into contact (mixed) with each other in order to avoid this red discoloration (for example, tranexamic acid and ascorbic acid should be formulated into separate granules) and conducted intensive studies.

However, if a mixture of tranexamic acid and ascorbic acid or a solid pharmaceutical preparation for oral administration obtained by formulating the mixture into a granule can be produced, the production time and labor would dramatically be saved. Therefore, it would be extremely useful.

That is, an object of the invention is to provide a pharmaceutical composition which is stable without causing discoloration or the like even if tranexamic acid and ascorbic acid are uniformly mixed and exist therein without separating tranexamic acid and ascorbic acid from each other by a boundary or the like.

### [Means to Solve the Problem]

As a result of studying preparations by repeating trials and errors to solve the above problems, the present inventors unexpectedly found surprising results that discoloration was prevented by adding a stabilizer which is a specified antioxidant or an organic acid other than ascorbic acid or tocopherol to a composition comprising tranexamic acid and ascorbic acid, and thus completed the present invention.

The invention is described as follows:
(1) A pharmaceutical composition comprising tranexamic acid and ascorbic acid and further comprising an organic acid or an antioxidant other than ascorbic acid or tocopherol as a stabilizer, wherein the components contained in the composition are uniformly mixed.
(2) A method of using an organic acid or an antioxidant other than ascorbic acid or tocopherol as a stabilizer for preventing discoloration of a composition caused by the presence of tranexamic acid and ascorbic acid.
(3) A method of preventing discoloration caused when tranexamic acid and ascorbic acid are incorporated in a same composition by adding an antioxidant or an organic acid other than ascorbic acid or tocopherol as a stabilizer to said composition.
(4) A method of producing a solid pharmaceutical preparation for oral administration, comprising steps of: mixing tranexamic acid and ascorbic acid; further adding and mixing an organic acid or an antioxidant other than ascorbic acid or tocopherol as a stabilizer; and granulating or compression-molding the resulting mixture.
(5) The pharmaceutical composition according to (1), wherein the organic acid serving as a stabilizer is at least one organic acid selected from citric acid, tartaric acid and malic acid.
(6) The pharmaceutical composition according to (1), wherein the antioxidant serving as a stabilizer is at least one antioxidant selected from sodium sulfite and sodium pyrosulfite.
(7) The method according to any one of (2) to (4), wherein the organic acid serving as a stabilizer is at least one organic acid selected from citric acid, tartaric acid and malic acid.
(8) The method according to any one of (2) to (4), wherein the antioxidant serving as a stabilizer is at least one antioxidant selected from sodium sulfite and sodium pyrosulfite.

### [Effect of the Invention]

According to the invention, even a solid pharmaceutical composition comprising tranexamic acid and ascorbic acid can be formulated into a single mixture or granule without formulating these components into separate granules. Further, the pharmaceutical composition of the invention is stable without causing discoloration and the like, and therefore, a solid pharmaceutical preparation for oral administration can be produced simply and at low cost.

### [Mode for Carrying Out the Invention]

Examples of the solid pharmaceutical preparation using the pharmaceutical composition of the invention include a granule, a pill, a powder, a tablet and a capsule listed in the Japanese Pharmacopoeia Fifteenth Edition. As the preparation, a granule or a tablet is preferred, and a sugar-coated granule, a film-coated granule, a sugar-coated tablet or a film-coated tablet is more preferred.

The "antioxidant" serving as the stabilizer to be used in the invention includes dried sodium sulfite, sodium hydrogen sulfite, sodium pyrosulfite, sodium nitrite, dibutyl hydroxy toluene, butyl hydroxy anisole and propyl gallate.

The "organic acid" serving as the stabilizer to be used in the invention includes anhydrous citric acid, citric acid hydrate, sodium citrate hydrate, tartaric acid, malic acid, succinic acid, gallic acid, acetic acid and lactic acid.

Tranexamic acid, ascorbic acid, an antioxidant (such as dried sodium sulfite, sodium hydrogen sulfite or sodium pyrosulfite) and an organic acid (such as anhydrous citric acid, citric acid hydrate or sodium citrate hydrate) to be used in the invention are listed in the Japanese Pharmacopoeia Fifteenth Edition.

Further, as for the components which are not listed in the Japanese Pharmacopoeia Fifteenth Edition, an ascorbate (such as sodium ascorbate or calcium ascorbate), an antioxidant (such as sodium nitrite, dibutyl hydroxy toluene, butyl hydroxy anisole or propyl gallate) and an organic acid (such as tartaric acid, malic acid, succinic acid, gallic acid, acetic acid or lactic acid) are commercially available and easily obtainable.

In the pharmaceutical composition of the invention, with respect to the content ratios of tranexamic acid, ascorbic acid and the stabilizer, based on 1 part by weight of tranexamic acid, ascorbic acid and the stabilizer are contained at 0.01 to 10 parts by weight and 0.001 to 1 part by weight, respectively, preferably at 0.1 to 5 parts by weight and 0.01 to 0.5 part by weight, respectively.

The pharmaceutical composition of the invention is characterized in that tranexamic acid and ascorbic acid stably exist therein without causing discoloration even if tranexamic acid and ascorbic acid are mixed with each other and exist therein by adding a stabilizer to a composition comprising tranexamic acid and ascorbic acid without resort to a boundary or the like.

Hereinafter, a method of producing a solid pharmaceutical preparation for oral administration of the invention is described.

The method of producing the preparation of the invention is characterized in that it comprises the steps of: mixing tranexamic acid, ascorbic acid and a stabilizer; and granulating or compression-molding the resulting mixture. For the mixing, granulating and compression-molding, known techniques can be used.

In the case where the preparation of the invention is a granule or a powder, it can be produced by mixing tranexamic acid, ascorbic acid and a stabilizer with pharmaceutical additives including an excipient such as crystalline cellulose, a disintegrant such as low-substituted hydroxypropyl cellulose and the like, and then, granulating the resulting mixture.

In the case where the preparation of the invention is a tablet, tranexamic acid, ascorbic acid and a stabilizer are mixed with pharmaceutical additives including an excipient such as crystalline cellulose, a disintegrant such as low-substituted hydroxypropyl cellulose and the like. The resulting mixture may be compression-molded as it is. Alternatively, after all the components are mixed, the resulting mixture is granulated, and then, the resulting granulated matter may be compression-molded.

Further, such a granule or a tablet can be formed into a film-coated granule, a sugar-coated tablet or a film-coated tablet through sugar coating or film coating by a known method.

Hereinafter, Examples are described for illustrating the invention in more detail.

### [Example 1]

14.0 g of tranexamic acid, 5.6 g of ascorbic acid and 0.4 g of sodium pyrosulfite were mixed in a mortar such that all the components were uniformly mixed. To this mixture, 1200 µL of purified water was added and the mixture was kneaded in a mortar, followed by granulating, thereby a granule was obtained.

### [Example 2]

13.0 g of tranexamic acid, 5.3 g of ascorbic acid and 1.7 g of sodium sulfite were mixed in a mortar such that all the components were uniformly mixed. To this mixture, 1200 µL of purified water was added and the mixture was kneaded in a mortar, followed by granulating, thereby a granule was obtained.

### [Example 3]

12.2 g of tranexamic acid, 4.9 g of ascorbic acid and 2.9 g of citric acid were mixed in a mortar such that all the components were uniformly mixed. To this mixture, 1200 µL of purified water was added and the mixture was kneaded in a mortar, followed by granulating, thereby a granule was obtained.

### [Example 4]

12.2 g of tranexamic acid, 4.9 g of ascorbic acid and 2.9 g of malic acid were mixed in a mortar such that all the components were uniformly mixed. To this mixture, 1200 µL of purified water was added and the mixture was kneaded in a mortar, followed by granulating, thereby a granule was obtained.

### [Example 5]

12.2 g of tranexamic acid, 4.9 g of ascorbic acid and 2.9 g of tartaric acid were mixed in a mortar such that all the components were uniformly mixed. To this mixture, 1200 µL of purified water was added and the mixture was kneaded in a mortar, followed by granulating, thereby a granule was obtained.

### [Comparative Example 1]

14.3 g of tranexamic acid and 5.7 g of ascorbic acid were mixed in a mortar such that all the components were uniformly mixed. To this mixture, 1200 µL of purified water was added and the mixture was kneaded in a mortar, followed by granulating, thereby a granule was obtained.

### [Comparative Example 2]

13.0 g of tranexamic acid, 5.3 g of ascorbic acid and 1.7 g of tocopherol acetate were mixed in a mortar such that all the components were uniformly mixed. To this mixture, 1200 µL of purified water was added and the mixture was kneaded in a mortar, followed by granulating, thereby a granule was obtained.

### [Preparation Example 1]

96.2 g of tranexamic acid, 38.5 g of ascorbic acid, 25.6 g of citric acid and an appropriate amount of crystalline cellulose were put into a fluidized bed granulator and mixed therein. Then, as a binder, an aqueous hydroxypropyl cellulose solution was sprayed, thereby a granulated granule was prepared. In 220.0 g of the granulated granule, 10.3 g of crystalline cellulose as an excipient, 5.1 g of croscarmellose sodium as a disintegrant, 2.1 g of magnesium stearate as a lubricant were mixed and the resulting mixture was tabletted, thereby an uncoated tablet serving as a core was prepared.

### [Preparation Example 2]

96.2 g of tranexamic acid, 38.5 g of ascorbic acid, 2.6 g of sodium pyrosulfite and an appropriate amount of crystalline cellulose were put into a fluidized bed granulator and mixed therein. Then, as a binder, an aqueous hydroxypropyl cellulose solution was sprayed, thereby a granulated granule was prepared. In 220.0 g of the granulated granule, 10.3 g of crystalline cellulose as an excipient, 5.1 g of croscarmellose sodium as a disintegrant, 2.1 g of magnesium stearate as a lubricant were mixed and the resulting mixture was tabletted, thereby an uncoated tablet serving as a core was prepared.

### (Test for Discoloration Prevention)

### 1.1. Test Method

Comparative Example 1 and Examples 1 to 6 were left under severe conditions (50°C, open, 2 days), and discoloration of each sample was visually observed.

With respect to Examples 1 to 6 and Comparative Example 1, a color difference ΔE between at the time of starting the test and at the time of stopping the test was measured using a spectroscopic colorimeter (SE-200, manufactured by NIPPON DENSHOKU INDUSTRIES CO., LTD.), and discoloration was quantitatively evaluated.

The results are shown in Table 1. Visual discoloration evaluation was performed according to the following criteria.

### (Evaluation Criteria)

Stable: A (no discoloration or slight discoloration) Somewhat Unstable: B (some discoloration)
Unstable: C (significant discoloration)

**[Table 1]**

| | Active ingredients | Stabilizer | Evaluation results 50°C, open, 2 days | |
|---|---|---|---|---|
| | | | Visual evaluation | ΔE |
| Example 1 | tranexamic acid + ascorbic acid | sodium pyrosulfite | A | 3.0 |
| Example 2 | tranexamic acid + ascorbic acid | sodium sulfite | A | 5.6 |
| Example 3 | tranexamic acid + ascorbic acid | citric acid | B | 10.3 |
| Example 4 | tranexamic acid + ascorbic acid | malic acid | B | 13.7 |
| Example 5 | tranexamic acid + ascorbic acid | tartaric acid | B | 15.4 |
| Comparative Example 1 | tranexamic acid + ascorbic acid | - | C | 19.8 |
| Comparative Example 2 | tranexamic acid + ascorbic acid | tocopherol acetate | C | 18.0 |

### 1.2. Test Results

From the results shown in Table 1, it was found that a solid pharmaceutical composition capable of remarkably preventing discoloration can be produced in the case where an antioxidant such as sodium pyrosulfite or an organic acid such as malic acid is added as a stabilizer to a composition in which tranexamic acid and ascorbic acid are uniformly present. In particular, when an antioxidant such as sodium pyrosulfite is used, an excellent effect can be exerted.

On the other hand, it was found that the effect of preventing discoloration of a tocopherol serving as an antioxidant is low.

## Claims

1. A solid pharmaceutical composition comprising tranexamic acid and ascorbic acid and further comprising an organic acid selected from the group consisting of citric acid, tartaric acid and malic acid or an antioxidant selected from the group consisting of sodium sulfite and sodium pyrosulfite as a stabilizer, wherein the components contained in the composition are uniformly mixed.

2. Use of an organic acid selected from the group consisting of citric acid, tartaric acid and malic acid or an antioxidant selected from the group consisting of sodium sulfite and sodium pyrosulfite as a stabilizer for preventing discoloration of a solid pharmaceutical composition caused by the presence of tranexamic acid and ascorbic acid.

3. A method of producing a solid pharmaceutical preparation for oral administration, comprising steps of: mixing tranexamic acid and ascorbic acid; further adding and mixing an organic acid selected from the group consisting of citric acid, tartaric acid and malic acid or an antioxidant selected from the group consisting of sodium sulfite and sodium pyrosulfite as a stabilizer; and granulating or compression-molding the resulting mixture.

4. The pharmaceutical composition according to claim 1, comprising the organic acid serving as a stabilizer.

5. The pharmaceutical composition according to claim 1, comprising the antioxidant as a stabilizer.

6. The use according to claim 2, wherein the organic acid serves as a stabilizer.

7. The use according to claim 2, wherein the antioxidant serves as a stabilizer.

8. The method according to claim 3, wherein the organic acid serves as a stabilizer.

9. The method according to claim 3, wherein the antioxidant serves as a stabilizer.

## Patentansprüche

1. Feste pharmazeutische Zusammensetzung, die Tranexaminsäure und Ascorbinsäure umfasst und weiterhin eine organische Säure, die aus der Gruppe bestehend aus Citronensäure, Weinsäure und Äpfelsäure ausgewählt ist, oder ein Antioxidationsmittel, das aus der Gruppe bestehend aus Natriumsulfit und Natriumpyrosulfit ausgewählt ist, als ein Stabilisierungsmittel umfasst, wobei die Bestandteile, die in der Zusammensetzung enthalten sind, gleichmäßig gemischt werden.

2. Verwendung einer organischen Säure, die aus der Gruppe bestehend aus Citronensäure, Weinsäure und Äpfelsäure ausgewählt ist, oder eines Antioxidationsmittel, das aus der Gruppe bestehend aus Natriumsulfit und Natriumpyrosulfit ausgewählt ist, als ein Stabilisierungsmittel zur Verhinderung der Verfärbung einer festen pharmazeutischen Zusammensetzung, die durch das Vorliegen von Tranexaminsäure und Ascorbinsäure verursacht wird.

3. Verfahren zur Herstellung eines festen pharmazeutischen Präparats zur oralen Verabreichung, das die folgenden Schritte umfasst: Mischen von Tranexaminsäure und Ascorbinsäure; weiterhin Zugeben und Mischen einer organischen Säure, die aus der Gruppe bestehend aus Citronensäure, Weinsäure und Äpfelsäure ausgewählt ist, oder eines Antioxidationsmittels, das aus der Gruppe bestehend aus Natriumsulfit und Natriumpyrosulfit ausgewählt ist, als ein Stabilisierungsmittel und Granulieren oder Formpressen des resultierenden Gemischs.

4. Pharmazeutische Zusammensetzung nach Anspruch 1, das die organische Säure umfasst, die als ein Stabilisierungsmittel dient.

5. Pharmazeutische Zusammensetzung nach Anspruch 1, das das Antioxidationsmittel als ein Stabilisierungsmittel umfasst.

6. Verwendung nach Anspruch 2, wobei die organische Säure als ein Stabilisierungsmittel dient.

7. Verwendung nach Anspruch 2, wobei das Antioxidationsmittel als ein Stabilisierungsmittel dient.

8. Verfahren nach Anspruch 3, wobei die organische Säure als ein Stabilisierungsmittel dient.

9. Verfahren nach Anspruch 3, wobei das Antioxidationsmittel als ein Stabilisierungsmittel dient.

## Revendications

1. Composition pharmaceutique solide comprenant de l'acide tranexamique et de l'acide ascorbique et comprenant en outre un acide organique choisi dans le groupe constitué par l'acide citrique, l'acide tartrique et l'acide malique ou un antioxydant choisi dans le groupe constitué par le sulfite de sodium et le pyrosulfite de sodium comme stabilisant, dans laquelle les composants contenus dans la composition sont mélangés de manière uniforme.

2. Utilisation d'un acide organique choisi dans le groupe constitué par l'acide citrique, l'acide tartrique et l'acide malique ou un antioxydant choisi dans le groupe constitué par le sulfite de sodium et le pyrosulfite de sodium comme stabilisant pour empêcher la décoloration d'une composition pharmaceutique solide provoquée par la présence d'acide tranexamique et d'acide ascorbique.

3. Procédé de production d'une préparation pharmaceutique solide destinée à une administration orale, comprenant les étapes consistant à : mélanger l'acide tranexamique et l'acide ascorbique ; ajouter et mélanger ensuite un acide organique choisi dans le groupe constitué par l'acide citrique, l'acide tartrique et l'acide malique ou un antioxydant choisi dans le groupe constitué par le sulfite de sodium et le pyrosulfite de sodium comme stabilisant ; et granuler ou mouler par compression le mélange obtenu.

4. Composition pharmaceutique selon la revendication 1, comprenant l'acide organique qui sert de stabilisant.

5. Composition pharmaceutique selon la revendication 1, comprenant l'antioxydant comme stabilisant.

6. Utilisation selon la revendication 2, dans laquelle l'acide organique sert de stabilisant.

7. Utilisation selon la revendication 2, dans laquelle l'antioxydant sert de stabilisant.

8. Procédé selon la revendication 3, dans lequel l'acide organique sert de stabilisant.

9. Procédé selon la revendication 3, dans lequel l'antioxydant sert de stabilisant.
